# EUROPEAN PATENT SPECIFICATION

(11) **EP 2 561 089 B1**
(45) Date of publication and mention of the grant of the patent: **04.06.2014**
(21) Application number: 11715451.8
(22) Date of filing: 20.04.2011
(51) Int. Cl.: C12Q 1/68, C12N 15/113, A61K 38/17

(54) **Method of determining the risk of survival of tumor cells in the bone marrow of a patient**
Verfahren zur Bestimmung des Risikos von überlebenden Tumorzellen im Knochenmark eines Patienten
Procédé pour déterminer le risque de survie de cellules cancéreuses dans la moelle d'un patient

(30) Priority: 20.04.2010 EP 10004180
(43) Date of publication of application: 27.02.2013
(73) Proprietor: Universitätsklinikum Hamburg-Eppendorf, 20246 Hamburg (DE)
(72) Inventor: WIKMAN, Harriet, 20248 Hamburg (DE); PANTEL, Klaus, 22457 Hamburg (DE); WERNER, Stefan, 22303 Hamburg (DE)
(74) Representative: UEXKÜLL & STOLBERG
(86) International application number: PCT/EP2011/002030
(87) International publication number: WO 2011/131360

(56) References cited:
- WO-A1-2009/124251
- WO-A1-2009/151914
- WO-A2-2007/109026
- US-A1- 2009 305 256
- ANONYMOUS: "GeneChip Human Genome Arrays", INTERNET CITATION, 2004, XP002384937,
- LEWIS M T ET AL: "Regulated expression patterns of IRX-2, an Iroquois-class homeobox gene, in the human breast.", CELL AND TISSUE RESEARCH JUN 1999 LNKD- PUBMED:10370142, vol. 296, no. 3, June 1999 (1999-06), pages 549-554, XP002587489, ISSN: 0302-766X

### SEQUENCE LISTING

<110> Universitaetsklinikum Hamburg-Eppendorf
<120> Method of determining the metastatic potential of a tumor
<130> P 82533
<160> 6
<170> PatentIn version 3.5
<210> 1
   <211> 19
   <212> DNA
   <213> Artificial Sequence
<220>
   <223> Primer
<400> 1
   ctcaatccga atggcaatg 19
<210> 2
   <211> 19
   <212> DNA
   <213> Artificial Sequence
<220>
   <223> Primer
<400> 2
   ctgaaagacg tgctgctcc 19
<210> 3
   <211> 22
   <212> DNA
   <213> Artificial Sequence
<220>
   <223> Primer
<400> 3
   gctaccagaa gcaaggacga ga 22
<210> 4
   <211> 21
   <212> DNA
   <213> Artificial Sequence
<220>
   <223> Primer
<400> 4
   agtgatccgt gagcgagtcc a 21
<210> 5
   <211> 21
   <212> DNA
   <213> Artificial Sequence
<220>
   <223> Primer
<400> 5
   acccagctct ggagaaactg c 21
<210> 6
   <211> 21
   <212> DNA
   <213> Artificial Sequence
<220>
   <223> Primer
<400> 6
   tgaggtcctc cttggtgaac a 21

## Description

The present invention relates to the field of tumor biology. In particular, it provides a method for determining the risk of survival of tumor cells in the bone marrow of a patient. The invention has a crucial impact on dissemination of tumor cells and survival of these cells in Bone marrow (BM) and/or distant organs as a prerequisite for metastatic relapse. The risk of survival of tumor cells in the bone marrow of a patient is assessed by determining the expression of RAI2, and optionally of other genes, in a tumor sample obtained from the patient. The invention further provides a kit for determining the expression of RAI2 and/or other genes. A pharmaceutical composition comprising RAI2 in gene or protein form is disclosed, in particular for preventing and/or treating metastasis of a tumor, and for thus preventing cancer-related death.

Solid tumors derived from epithelial tissues, i.e., carcinomas, are the major form of cancer in industrialized countries with approximately 380.000 new cases each year in Germany. A key event in the progression of these tumors is the systemic spread of tumor cells from the primary lesion through the blood circulation into distant organs. Metastasis, the main cause of cancer-related deaths, is a complex multi-step process. The onset of this critical process, i.e., dissemination of single tumor cells, remains undetected even by high-resolution imaging technologies, because these technologies are unable to detect metastasizing cells at the single cell level (Pantel et al., 2008). The inventors and other researchers have shown that the presence of disseminated tumor cells (DTC) is an independent predictor of metastatic relapse in breast cancer (Braun et al., 2005) and other solid tumors (Köllermann et al., 2008; Dango et al., 2008). These DTC can survive chemotherapy and persist in the BM over many years in a "dormant" state (Janni et al., 2005), suggesting that the BM might be a reservoir for metastatic cells (Wikman et al., 2008; Pantel et al., 2008). So far, it is not clear whether it is *de novo* mutations in the DCT or signals from the surrounding environment that make these cells escape the dormant state leading to proliferation and formation of overt metastasis (Pantel et al., 2008; Wikman et al., 2008). It has been shown that most early disseminated cancer cells detected in bone marrow of breast cancer patients have a CD44(+) and CD24(-/low) phenotype. This putative breast cancer stem cell phenotype is present in a minor population of primary breast cancer which is associated with self-renewal and tumorigenic potential (Balic et al., 2006).

So far, several important oncogenes and tumor suppressor genes (TSGs) are well known for breast cancer formation, including HER2, p53 and EGFR. However, much less is known about the specific role of genes which exclusively promote or inhibit crucial steps of the metastatic cascade. Among these the metastasis suppressor genes (MSGs), by definition, inhibit metastasis at any step of the metastatic cascade without blocking primary tumor growth (Stafford et al., 2008). As they encode for proteins with a wide range of biochemical activities, this class of genes is only unified by their capacity to suppress metastasis. The expanding list of metastasis suppressors have been shown to work by regulating signaling pathways and by other molecular functions as diverse as adhesion migration, apoptosis and angiogenesis (Stafford et al., 2008). In addition, MSGs have an impact upon different stages of the metastatic cascade to manifest their suppressive effects. Some MSGs, like E-Cadherin or Gelsolin inhibit tumor cell motility and invasion in the primary tumor, while others, like DCC and Caspase-8, repress cell survival in the circulation (Eccles et al., 2007). Other MSGs have been implicated to function in tumor dormancy at the secondary site and thereby regulate the final stage of metastasis. However, since metastasis is the major cause of cancer-related deaths and every step in the metastatic cascade is considered rate-limiting (Pantel and Brakenhoff, 2004), the reconstitution of a gene or protein that inhibits any step in the cascade should provide a promising therapeutically window for the treatment of cancer patients.

Although targeting a negative regulator is technically challenging, recent works have successfully reinduced the function of MSGs, administered the gene product itself (Taneka et al. 2007) or targeted druggable molecules regulated by the suppressor protein (Boucharaba 2006). Although none of these strategies are yet in routine clinical use, they are being tested preclinically and in clinical trials. Accordingly, the identification of additional MSGs could lead to further promising metastasis suppressor-based strategies.

New techniques for the enrichment, detection and characterization of disseminating tumor cells in the bone marrow and the peripheral blood have been developed. Single disseminated tumor cells (DTC) can be detected before the background of millions of normal bone marrow or blood cells. Using immunocytochemical double staining techniques, the expression patterns of different proteins in DTC have been described, including Ki-67, p120, EGFR, Her2, transferring-R, EMMPRIN, uPAR and EpCam (Pantel and Brakenhoff, 2008).

Most metastasis suppressor genes exhibit decreased expression in highly metastatic primary tumors as compared with non-metastatic tumors. Thus, screening experiments to identify metastasis suppressors can be carried out by comparing cells or tissues of different metastatic competence. In a small pilot study, it could be shown that distinct profiles exist between primary tumors from BM-positive and BM-negative patients (Wölfle et al., 2003). The BM signature was mainly characterized by transcriptional repression, and it is different from the expression signature associated with lymphatic metastasis. BM micrometastasis thus is a selective process with a specific molecular signature of the primary tumor (Wölfle et al., 2003). Recently, a gene expression signature for early tumor dissemination in lung cancer was described, wherein five chromosomal regions differentiating patients with DTC in the BM from patients without such cells at time of primary surgery were defined (Wrage et al., 2009).

In light of the state of the art, the inventors addressed the problem of providing a novel marker for the risk of survival of tumor cells in bone marrow as key steps in the metastasic progression of a tumor. The inventors further addressed the problem of providing a set of markers useful for assessing the risk of said survival. The identified marker can also be used as a target in therapeutic approaches to cancer.

The present invention provides a method of determining the risk of survival of tumor cells in the bone marrow of a patient, wherein the expression of RAI2 (retinoid acid induced gene 2) in a tumor sample obtained from the patient is determined.

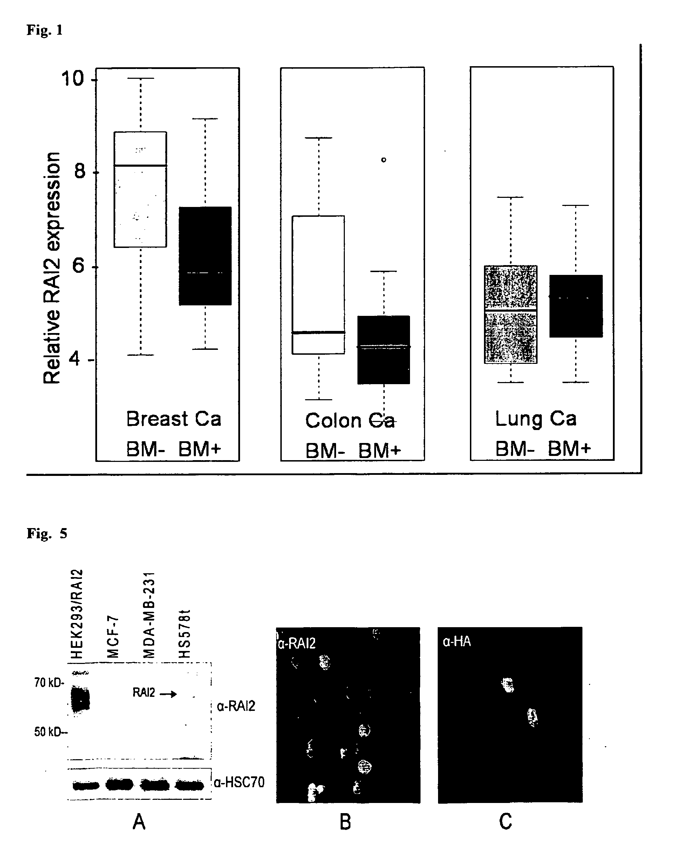

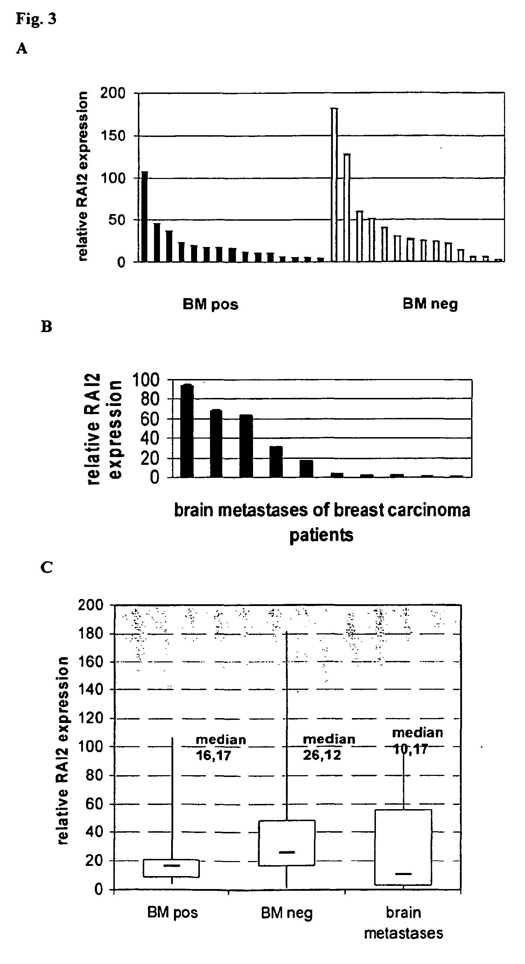

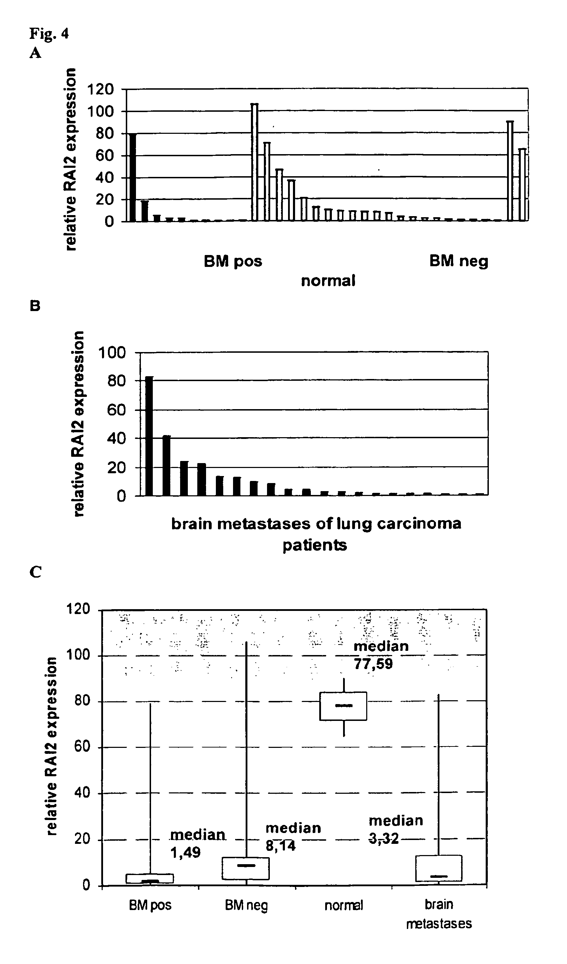

The inventors found that a low expression of RAI2, a gene previously hardly analyzed at all, indicates a high risk of metastasis, in particular early tumor cell metastasis events such as dissemination of tumor cells (Fig. 1). In DTC-positive patients, i.e., in patients with a high risk of metastatic progression, expression of RAI2 was shown to be very low in tumor tissue in comparison to e.g. normal lung and imortilized normal epithelial breast and lung cancer cell lines as determined by quantitive RT-PCR (Fig. 3, 4, 15, Table B). The inventors have found that analysis of RAI2 expression alone, as a factor independent from expression of other genes, allows important predictions. While formation of metastasis is of course critical for a patient's survival versus cancer-related death, by assessing the risk of survival in bone marrow, the invention makes it possible to look at key steps in the metastasic progression of a tumor, which allows a much earlier intervention than looking at factors only associated with overall survival or relapse of a patient.

Expression of RAI2 in a patient sample can be assessed as "low" if it is lower than expression in standard cell lines (e.g. commercially avalaible immortalized normal human epithelial breast and lung cell lines MCF10A and BEAS2B), or than expression in an average tissue sample of sampe origin from a healthy subject of the same species as the patient. For comparison, Universal Human Reference, UHR, can be used as a reference. Assessment of the RT-PCR data can be carried out by normalization against expression of the reference gene RPLPO (60S acidic ribosomal protein P0) in the same sample using the ΔΔCₜ method. (Methods of data assessment can be carried out as taught in Molloy et al., Breast Cancer Res Treat. 2008 Nov;112(2):297-307). The lower the expression of RAI2 in the sample from the patient, the higher is the risk of survival of tumor cells in the bone marrow.

In a gene expression profiling study covering the whole genome, the inventors defined individual genes associated with the BM status. By using gene expression profiling on primary breast, colorectal and lung tumors, they were able to identify genes associated with BM status. Remarkably, it was found that most genes with significant expression differences between DTC-positive and DTC-negative patients are down regulated in the DTC-positive group, which would argue for an important role of progression/dissemination suppressors instead of activators.

The inventors found that one of those metastasis suppressors is the retinoid acid induced gene 2 (RAI2). RAI2 was found to be significantly down regulated in primary tumors of BM positive patients in the breast cancer study as well as in the colorectal cancer study, and in the overall analysis. The summary of *RAI2* expression data in bone marrow positive and negative patients from the RNA expression profiling in different tumor entities is shown in Fig. 1. *RAI2* represents an almost uncharacterized gene, and the specific molecular function of the corresponding gene product has not yet been determined. The murine RAI2 gene was first described by Jonk et al., (1994) who observed the induction of the Rai2 transcript in murine embryonic carcinoma P19 cells after treatment with all-trans retinoic acid (ATRA). The orthologic human RAI2 gene is located at Xp22.3 and was first cloned and described ten years ago by Walpole et al.. It was shown that the RAI2 transcript is expressed in several fetal and adult tissues (Walpole et al., 1999).

Interestingly, recently Finak et al. (2008) compared gene expression profiles of breast tumor stroma and described stroma-derived prognostic predictor that stratified disease outcome independently of standard clinical prognostic factors. *RAI2* was described as one of the 163 tumor stromal genes which could predict clinical outcome in breast cancer as determined by relapse-free survival (Finak et al., 2008). The importance of RAI2 as single gene within this signature was not further evaluated in this or any later reports, and RAI2 was therefore not highlighted as an important predictor of metastasis. Moreover, the present inventors' analysis, used macro-dissected tumor tissue and thus indicates that *RAI2* is also an important gene in the tumors and not only in the stroma, and it allows assessing the risk of metastasis, i.e., an early step in the progression of cancer compared to survival. In some previous expression analysis, RAI2, among an extensive list of other genes, was described as a gene upregulated in cancer (US application No. 20090175844, US application No. 20080318234, US application No. 20080292546). While this does not necessarily have to be contradictory to the teaching of present invention, it is for the first time shown herein that the downregulation of RAI2 in tumor tissue allows assessment of the risk of metastasis or the tumor.

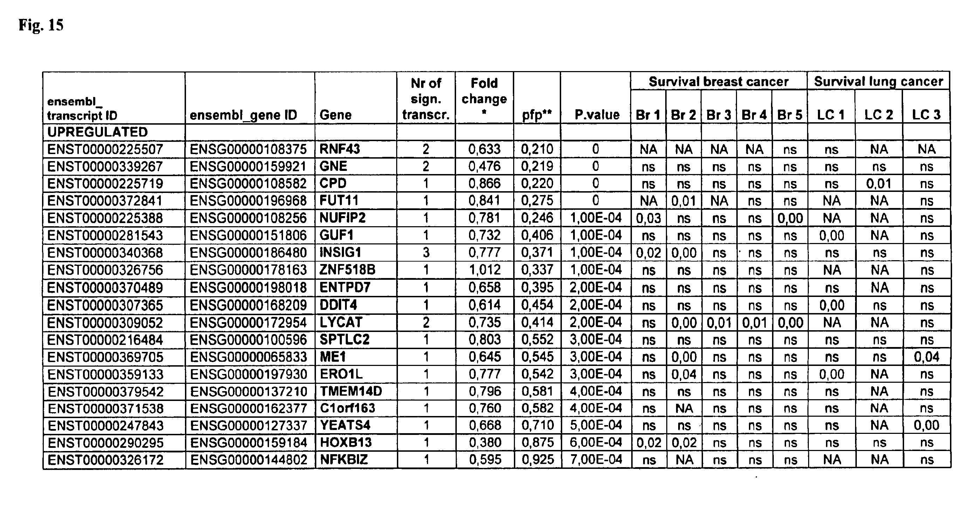

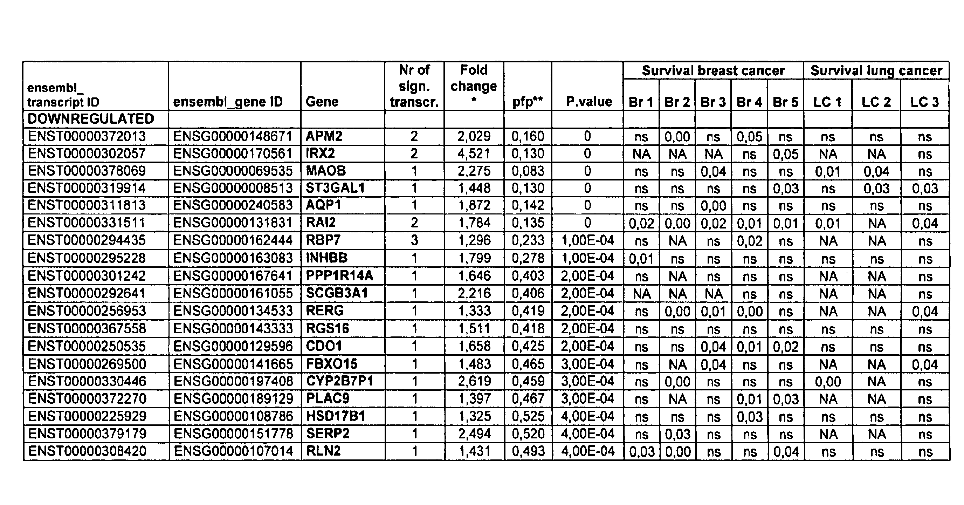

A summary of up- and downregulated genes among BM positive patients of combined analysis of tumors from breast, lung and colorectum, is shown in Fig. 15.

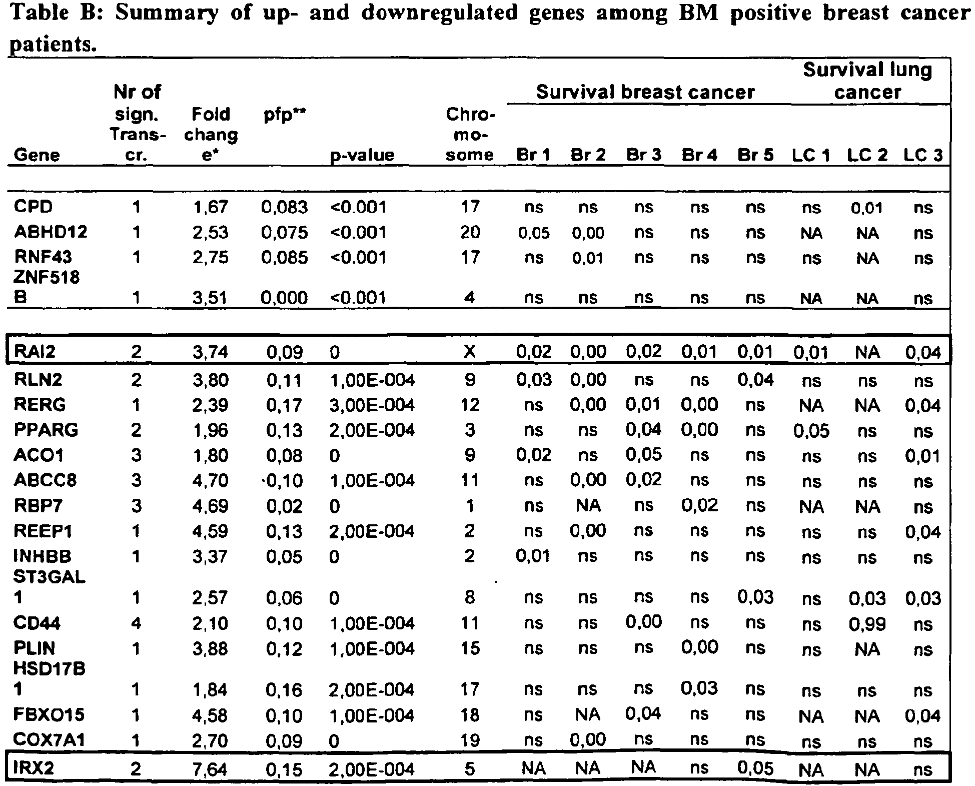

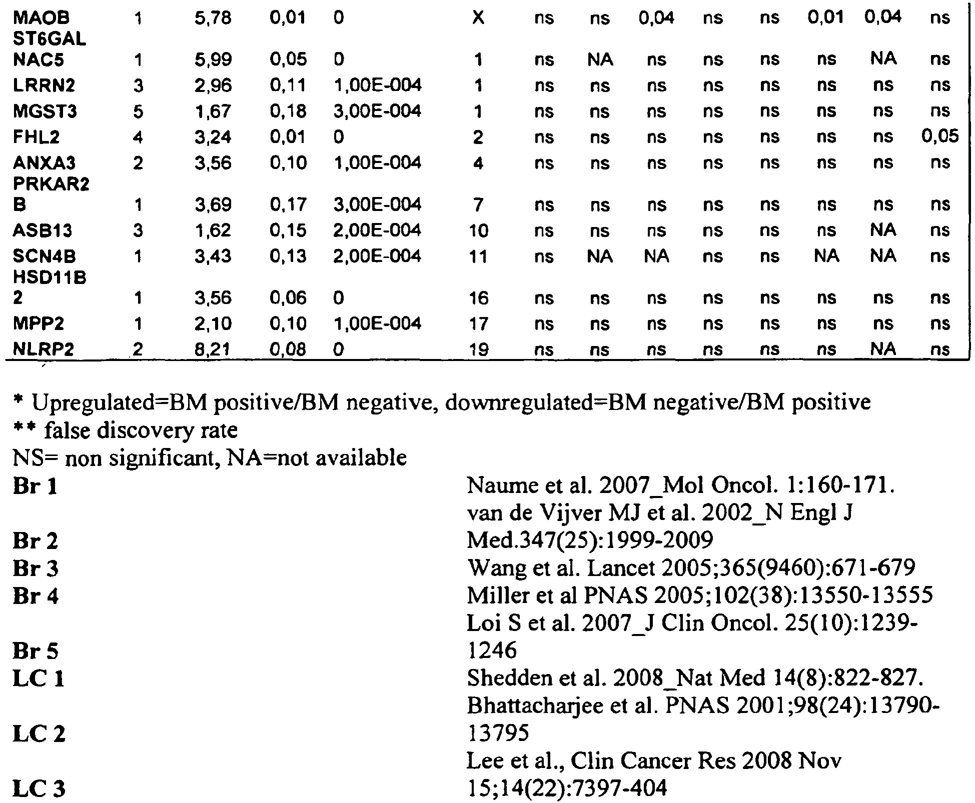

The sequences of the transcripts are disclosed in the database Ensembl protein_coding Genes (www.ensembl.org) and can be identified by their unique identifiers specified in the following table.

**Table C: Ensembl IDs of relevant genes (www.ensembl.org) version GRCh37/hg19**

| **ensembl_genID** | **ensembl_transcript ID** | **Gene** | **Combined analysis** | **Breast ca. alone** |
|---|---|---|---|---|
| ENSG00000148671 | ENST00000372013 | **APM2** | down reg. | ns |
| ENSG00000240583 | ENST00000311813 | **AQP1** | down reg. | ns |
| ENSG00000129596 | ENST00000250535 | **CDO1** | down reg. | ns |
| ENSG00000197408 | ENST00000330446 | **CYP2B7P1** | down reg. | ns |
| ENSG00000189129 | ENST00000372270 | **PLAC9** | down reg. | ns |
| ENSG00000167641 | ENST00000301242 | **PPP1R14A** | down reg. | ns |
| ENSG00000143333 | ENST00000367558 | **RGS16** | down reg. | ns |
| ENSG00000161055 | ENST00000292641 | **SCGB3A1** | down reg. | ns |
| ENSG00000151778 | ENST00000379179 | **SERP2** | down reg. | ns |
| ENSG00000141665 | ENST00000269500 | **FBX015** | down reg. | down reg. |
| ENSG00000108786 | ENST00000225929 | **HSD17B1** | down reg. | down reg. |
| ENSG00000163083 | ENST00000295228 | **INHBB** | down reg. | down reg. |
| ENSG00000170561 | ENST00000302057 | **IRX2** | down reg. | down reg. |
| ENSG00000069535 | ENST00000378069 | **MAOB** | down reg. | down reg. |
| ENSG00000131831 | ENST00000331511 | **RAI2** | down reg. | down reg. |
| ENSG00000162444 | ENST00000294435 | **RBP7** | down reg. | down reg. |
| ENSG00000134533 | ENST00000256953 | **RERG** | down reg. | down reg. |
| ENSG00000107014 | ENST00000308420 | **RLN2** | down reg. | down reg. |
| ENSG00000008513 | ENST00000319914 | **ST3GAL1** | down reg. | down reg. |
| ENSG00000006071 | ENST00000302539 | **ABCC8** | ns | down reg. |
| ENSG00000122729 | ENST00000379921 | **ACO1** | ns | down reg. |
| ENSG00000138772 | ENST00000264908 | **ANXA3** | ns | down reg. |
| ENSG00000196372 | ENST00000357700 | **ASB13** | ns | down reg. |
| ENSG00000026508 | ENST00000263398 | **CD44** | ns | down reg. |
| ENSG00000161281 | ENST00000292907 | **COX7A1** | ns | down reg. |
| ENSG00000115641 | ENST00000322142 | **FHL2** | ns | down reg. |
| ENSG00000176387 | ENST00000326152 | **HSD11B2** | ns | down reg. |
| ENSG00000170382 | ENST00000367175 | **LRRN2** | ns | down reg. |
| ENSG00000143198 | ENST00000367883 | **MGST3** | ns | down reg. |
| ENSG00000108852 | ENST00000377184 | **MPP2** | ns | down reg. |
| ENSG00000022556 | ENST00000263437 | **NLRP2** | ns | down reg. |
| ENSG00000166819 | ENST00000300055 | **PLIN** | ns | down reg. |
| ENSG00000132170 | ENST00000287820 | **PPARG** | ns | down reg. |
| ENSG00000005249 | ENST00000265717 | **PRKAR2B** | ns | down reg. |
| ENSG00000068615 | ENST00000165698 | **REEP1** | ns | down reg. |
| ENSG00000177098 | ENST00000324727 | **SCN4B** | ns | down reg. |
| ENSG00000117069 | ENST00000318803 | **ST6GALNAC5** | ns | down reg. |
| ENSG00000162377 | ENST00000371538 | **C1orf163** | up reg. | ns |
| ENSG00000168209 | ENST00000307365 | **DDIT4** | up reg. | ns |
| ENSG00000198018 | ENST00000370489 | **ENTPD7** | up reg. | ns |
| ENSG00000197930 | ENST00000359133 | **ERO1L** | up reg. | ns |
| ENSG00000196968 | ENST00000372841 | **FUT11** | up reg. | ns |
| ENSG00000159921 | ENST00000339267 | **GNE** | up reg. | ns |
| ENSG00000151806 | ENST00000281543 | **GUF1** | up reg. | ns |
| ENSG00000159184 | ENST00000290295 | **HOXB13** | up reg. | ns |
| ENSG00000186480 | ENST00000340368 | **INSIG1** | up reg. | ns |
| ENSG00000172954 | ENST00000309052 | **LYCAT** | up reg. | ns |
| ENSG00000065833 | ENST00000369705 | **ME1** | up reg. | ns |
| ENSG00000144802 | ENST00000326172 | **NFKBIZ** | up reg. | ns |
| ENSG00000108256 | ENST00000225388 | **NUFIP2** | up reg. | ns |
| ENSG00000100596 | ENST00000216484 | **SPTLC2** | up reg. | ns |
| ENSG00000137210 | ENST00000379542 | **TMEM14D** | up reg. | ns |
| ENSG00000127337 | ENST00000247843 | **YEATS4** | up reg. | ns |
| ENSG00000108582 | ENST00000225719 | **CPD** | up reg. | up reg. |
| ENSG00000108375 | ENST00000225507 | **RNF43** | up reg. | up reg. |
| ENSG00000178163 | ENST00000326756 | **ZNF518B** | up reg. | up reg. |
| ENSG00000100997 | ENST00000376542 | **ABHD12** | ns | up reg. |

| | | | | |
|---|---|---|---|---|
| ns= not significant | | | | |

In one embodiment of the invention, expression of RAI2 and IRX2 (Iroquois homeobox 2) in the sample is determined. Alternatively, only expression of IRX2 in the sample may be determined, e.g., using the methods described for RAI2. The inventors found that a low expression of IRX2 independently indicates a high risk of metastasis.

In one embodiment of the invention, the expression of one or more genes selected from the group comprising APM2, AQP1, CDO1, CYP2B7P1, PLAC9, PPP1R14A, RGS16, SCGB3A1, SERP2, FBXO15, HSD17B1, INHBB, IRX2, MAOB, RBP7, RERG, RLN2, ST3GAL1, ABCC8, ACO1, ANXA3, ASB13, CD44, COX7A1, FHL2, HSD11B2, LRRN2, MGST3, MPP2, NLRP2, PLIN, PPARG, PRKAR2B, REEP1, SCN4B, ST6GALNAC5, C1orf163, DDIT4, ENTPD7, ERO1L, FUT11, GNE, GUF1, HOXB13, INSIG1, LYCAT, ME1, NFKBIZ, NUFIP2, SPTLC2, TMEM14D, YEATS4, CPD, RNF43, ZNF518B, and ABHD12 in the sample is determined, in addition to expression of RAI2, or expression of all af these genes is determined. Expression or two, three, four, five, six, seven, eight, nine, ten or more of these genes may be determined. Preferably, for a given tumor type, genes found to be significantly upregulated or downregulated, as disclosed in the tables above, are analyzed. For example, for analysis of breast cancer, expression of one or more of FBXO15, HSD17B1, INHBB, IRX2, MAOB, RBP7, RERG, RLN2, ST3GAL1, ABCC8, ACO1, ANXA3, ASB13, CD44, COX7A1, FHL2, HSD11B2, LRRN2, MGST3, MPP2, NLRP2, PLIN, PPARG, PRKAR2B, REEP1, SCN4B, ST6GALNAC5, CPD, RNF43, ZNF518B, and ABHD12 in addition to RAI2, may be analysed, in one option, all of these. For analysis of colorectal cancer, expression of FAM3B, ARHGAP6, SERP2, FTSJ1, GEMIN8, NETO2, HOXB13, PDE9A and NEDD4L may be analysed in addition to RAI2. Of course, at the same time, expression of other genes can also be determined, or only the defined genes can be analyzed.

The tumor from which the sample is obtained preferably is a primary tumor, but it may also be a metastasis or disseminated tumor cells isolated from the patient. Expression may also be analysed in both the primary tumor and one or more metastases. The tumor may be any tumor of epithelial origin, i.e., a carcinoma. It may be selected from the group comprising breast cancer, lung cancer, or ovarian cancer and colorectal carcinoma. Preferably, the tumor is breast, lung, or colorectal cancer, most preferably, breast cancer. The sample may, e.g., be obtained by biopsy or during removal of the tumor. In one embodiment, the sample essentially consists of tumor cells, i.e., at least 50%, preferably, at least 60%, at least 70%, at least 80% or at least 90% of the cells of the sample are tumor cells. Such a sample can, e.g., be obtained by macrodissection or microdissection of the tumor.

The patient may be a human patient who has been diagnosed with a tumor. Alternatively, the patient may be a mouse, rabbit, rat, guinea pig, pig, sheep, goat, cattle, horse, dog, cat, monkey or ape. Of course, if the patient is not human, the expression of the respective RAI2 gene is tested. The identifiers described above relate to the human genes, and the respective animal homologues can be identified by the skilled person.

The expression of RAI2 may be determined on the protein or RNA level, e.g., using by RT-PCR, gene chip analysis, or hybidization. In one embodiment, RNA isolated from the sample is converted into cDNA using standard methods. Real time PCR (e.g., TAQman® or Lightcycler ® based methods) may be used for specific quantification of nucleotides. In one embodiment, the expression is analysed by means of a gene chip comprising a probe for RAI2, e.g., from Affymetrix, Santa Clara, CA. Expression of several genes can be analysed with a gene chip at the same time. For hybridization, e.g., RNA isolated from the sample or cDNA can be used. Examplary methods, e.g., primers or gene chips that may be used, are disclosed in the examples.

If expression is analyzed on the protein level, e.g., ELISA, Western Blot, dot blot, immunohistology, e.g, immunochemistry or immunofluorescent methods, or FACS may be used. A commercial polyclonal anti-RAI2-antibody is available from Abgent, San Diego, CA. Of course, other polyclonal or monoclonal antibodies may also be used. Histologic methods have the advantage that parafin-embedded tissue from classical histological examination of the biopsy can be used without need for further measures. There are also methods known in the art to enable processing of RNA from such samples, but analysis of the RNA is easier if the sample is used without sample treatment e.g. with parafin.

The invention also provides a kit for determining the risk of survival of tumor cells in the bone marrow of a patient, comprising means for determining the expression of RAI2 in a sample obtained from the patient by the method of the invention.

The means may, e.g., be selected from the group comprising an antibody to RAI2, primers capable of specifically hybridizing to and amplifying RAI2, and a probe capable of specifically hybridizing to RAI2. Selection of suitable probes and primers for the methods of detection described above are routine measures for the skilled person. Exemplary primers are also shown in the examples. The kit preferably also comprises instructions for use in determining the risk of a patient. The kit optionally comprises further suitable reagents, e.g., buffers.

Preferably, the kit comprises means for analysis of RAI2, APM2, AQP1, CDO1, CYP2B7P1, PLAC9, PPP1R14A, RGS16, SCGB3A1, SERP2, FBXO15, HSD17B1, INHBB, IRX2, MAOB, RBP7, RERG, RLN2, ST3GAL1, ABCC8, ACO1, ANXA3, ASB13, CD44, COX7A1, FHL2, HSD11B2, LRRN2, MGST3, MPP2, NLRP2, PLIN, PPARG, PRKAR2B, REEP1, SCN4B, ST6GALNAC5, C1orf163, DDIT4, ENTPD7, ERO1L, FUT11, GNE, GUF1, HOXB13, INSIG1, LYCAT, ME1, NFKBIZ, NUFIP2, SPTLC2, TMEM14D, YEATS4, CPD, RNF43, ZNF518B, and ABHD12. In one embodiment, the kit does not comprise means for analysis of other genes, except for, optionally, one, two, three or four control genes, such as RPLP0 (60S acidic ribosomal protein P0), ACTB (beta-actin), A2M (alpha-2-macroglobulin) and GAPDH (glyceraldehyde-3-phosphate dehydrogenase).

By systematic mapping of protein-protein interactions using stringent high-throughput yeast two-hybrid systems, it has been shown that the RAI2 gene product is an interacting molecule of the CTBP2 and the SGTB protein (Rual et al. 2005). While the molecular function of SGTB has not been described so far, CTBP2 represents a well characterized protein, which has long been known to counteract several tumor suppressor genes (Chinnadurai 2009). The CTBP2 gene product acts in the nucleus as a scaffolding protein for transcriptional repressor complexes, indicating that the RAI2 protein might be involved in transcriptional regulation (Chinnadurai 2009). Experiments performed by the inventors are in line with this hypothesis, showing, e.g., nuclear localization.

The inventors also showed that knock-down of RAI2 using siRNA may affect the cells' capability of undergoing apoptosis and lead to resistence to apoptosis.

As sensitivity to apoptosis is essential for chemotherapeutic treatment, also disclosed is a method of determining a patient's sensitivity to chemotherapy, comprising determining the expression of RAI2 in a tumor sample obtained from the patient, wherein a low expression of RAI2 indicates a low sensitivity to chemotherapy. The method can be carried out in analogy to the method described above.

The functional link between the downregulation of RAI2 in tumor cells and tumor formation and metastasis furthermore opens up a novel therapeutic approach. Disclosed is a pharmaceutical composition comprising RAI2 protein, a gene transfer vector comprising RAI2 functionally linked to a promoter or a compound capable of upregulating expression of RAI2. The pharmaceutical composition comprises an agent capable of providing RAI2 in the tumor cell. Thus, the pharmaceutical composition may be effective at least partially to compensate for the loss of RAI2 expression in tumor tissue. This may make the tumor more sensitive to apoptosis and/or reduce the risk of metastasis.

In one gene therapy, where a gene transfer vector comprising RAI2 functionally linked to a promoter is used, the promotor is an inducible promotor. This may be an inducible promotor active in tumor cells. The promotor may alternatively be a constitutive promotor. For example, the expression construct disclosed in the examples may be used. An exemplary compound capable of upregulating expression of RAI2 may be retinoic acid. An amount of retinoic acid sufficient to induce significant expression of RAI2 may already be sufficient to reduce the risk of metastasis of a tumor.

The pharmaceutical composition may be for use in preventing metastasis of a tumor and/or treating a tumor, e.g., a primary tumor and/or metastasis. Also disclosed is a method of preventing metastasis of a tumor and/or treating a tumor, comprising upregulating expression of RAI2 in the tumor and/or administering RAI2 to the tumor. The expression of RAI2 may be upregulated by administration of a gene transfer vector comprising RAI2 functionally linked to a promoter, e.g., as described above. RAI2 may be administered in gene or protein form. Reducing the risk of metastasis reduces the risk of cancer-related death of the patient. In particular, as discussed above, prevention of metastasis is the prevention of dissemination of tumor cells, which can be reduced by the disclosed method and the pharmaceutical composition.

In one embodiment, it is first tested if a patient's tumor expresses low levels of RAI2. In this case, the patient is to be treated with the disclosed pharmaceutical composition, in order to prevent metastasis of a tumor and/or treat a tumor.

Methods of targeting a tumor or metastasis suppressor gene have been described in the art. For example, the methods described in Palmieri, et al. 2005; Li et al. 2006; Taneka et al. 2007; Titus et al., 2005; Boucharaba 2006, Pantel and Brakenhoff et al., 2008 may be used analogically.

Also disclosed is a pharmaceutical composition comprising IRX2 protein, a gene transfer vector comprising IRX2 functionally linked to a promoter or a compound capable of upregulating expression of IRX2. The pharmaceutical composition comprises an agent capable of providing IRX2 in the tumor cell. The pharmaceutical composition may be for use in preventing metastasis of a tumor and/or treating a tumor, e.g., a primary tumor and/or metastasis. Also disclosed is a method of preventing metastasis of a tumor and/or treating a tumor, comprising upregulating expression of IRX2 in the tumor and/or administering IRX2 to the tumor. The expression of IRX2 may be upregulated by administration of a gene transfer vector comprising IRX2 functionally linked to a promoter, e.g., as described above. IRX2 may be administered in gene or protein form. It may be first tested if a patient's tumor expresses low levels of IRX2. In this case, the patient is to be treated with the disclosed pharmaceutical composition, in order to prevent metastasis of a tumor and/or treat a tumor.

The pharmaceutical composition may also comprise both RAI2 and IRX2. The pharmaceutical composition may also be used in combination with conventional treatments, such as chemotherapy, raditation therapy, and/or surgical removal of the primary tumor and/or metastases.

The pharmaceutical composition comprising a gene transfer vector comprising RAI2 functionally linked to a promoter or a compound capable of upregulating expression of RAI2 may also be used to induce senescence in tumor cells. Preferably, the gene transfer vector may be used in the pharmaceutical composition.

In the following, the invention is illustrated by examples. These are not to be construed as limiting the invention. Modifications and adaptions will be obvious to the skilled person.

### Figure legends

**Fig. 1****: Relative RAI2 expression in primary tumors of different tumor entities** from BM-positive and BM-negative patients. RAI2 expression in different groups is illustrated in Box-Whisher-Plots.
**Fig. 2****: Survival analysis in breast, lung cancer and ovarian data sets stratified by the RAI2 gene expression.** Samples in the data sets were separated into high expression and low expression groups of RAI2 and Kaplan-Meier estimates were performed. Upper line: highest quartile, lower line: lowest quartile.
**Fig. 3****: Analysis of *RAI2* Expression in breast cancer samples** by quantitative RT-PCR. Relative RAI2 transcript levels were determined by normalized to the reference gene RPLP0 (60S acidic ribosomal protein P0) using the ΔΔCₜ method. Groups are illustrated in Box-Whisher-Plots (upper quartile is not completely displayed).
**Fig. 4****: Analysis of *RAI2* Expression in lung cancer samples** by quantitative RT-PCR (as described for Fig. 3).
**Fig. 5****: Analysis of RAI2 protein expression.** A. Western Blot analysis of RAI2 protein expression in whole cell extracts using polyclonal anti-RAI2-anitbody (Abgent). B and C. Immunocytochemical detection of RAI2 in HEK293 cells with polyclonal anti-RAI2-anitbody (B) (Abgent, San Diego, CA) and monoclonal anti-HA antibody (C) (Sigma-Aldrich, Steinheim). Nuclear staining was performed using DAPI dye (blue) and merged with protein specific staining (green fluorescence).

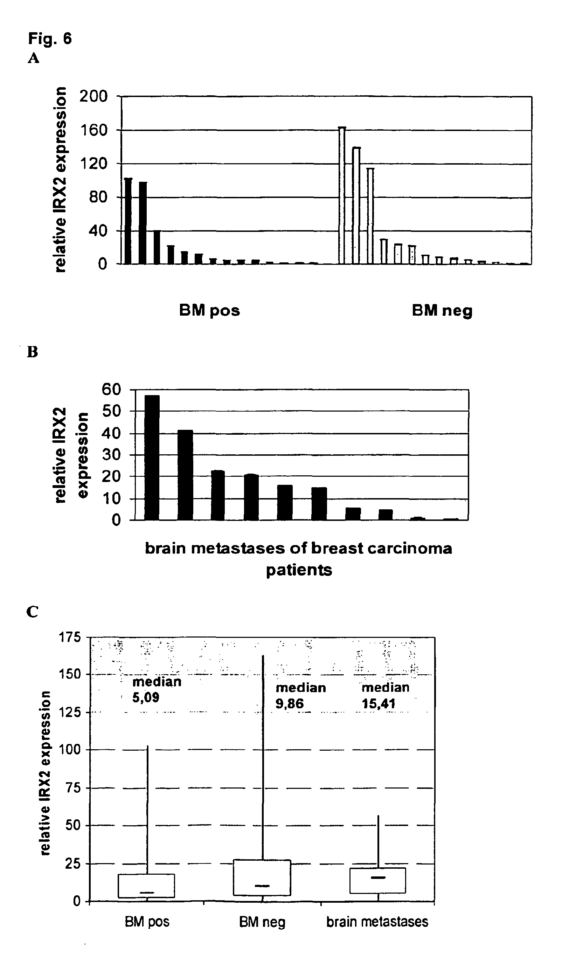

**Fig. 6****: Relative IRX2 expression in breast tumor samples** by quantitative RT-PCR (as described for Fig. 3).

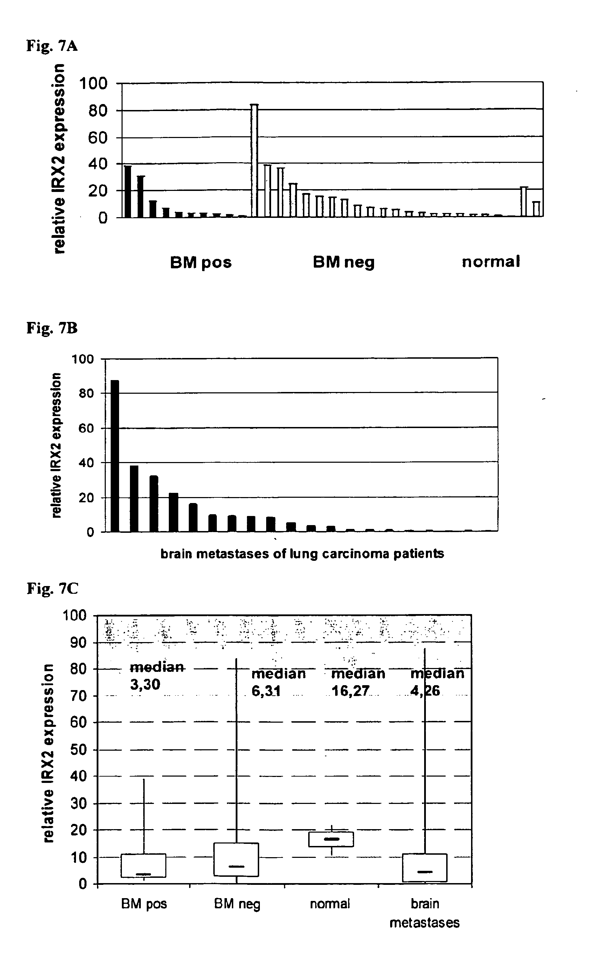

**Fig. 7****: Relative IRX2 expression in lung carcinoma samples** by quantitative RT-PCR (as described for Fig. 3).

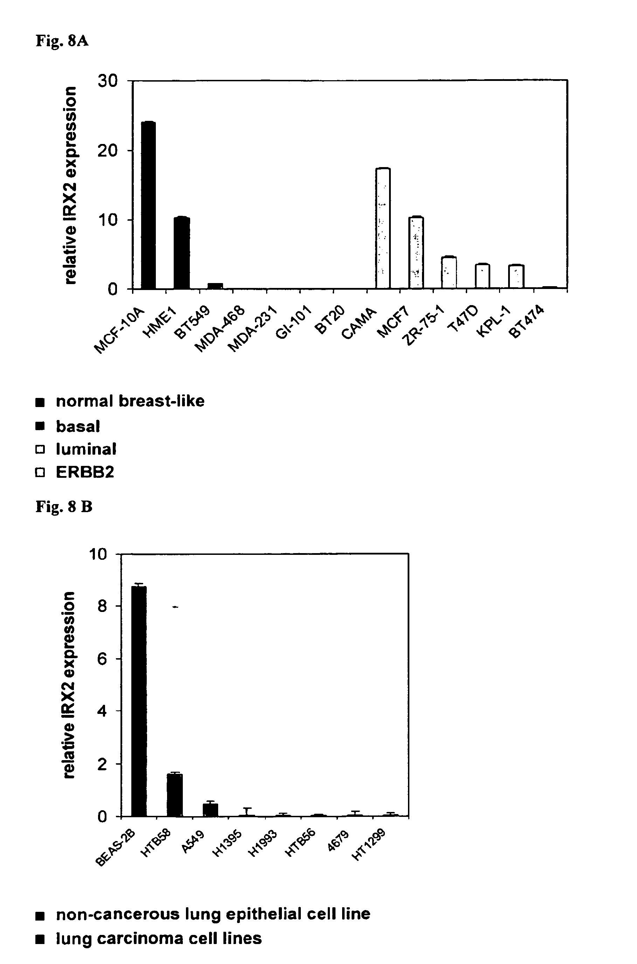

**Fig. 8****: Relative IRX2 expression in cancer cell lines** by quantitative RT-PCR (as described for Fig. 3).
**Fig. 9****: Analysis of *RAI2* expression in MDA-MB-231 breast cancer cells after treatment with the indicated DNA damage and cellular stress inducing compounds.** Relative RAI2 transcript levels were determined by normalizing to the reference gene RPLPO using the ΔΔCₜ method.
**Figure 10****: Analysis of *RAI2* transcript levels in RAI2-shRNA transduced MDA-MB-231 and MCF-7 breast cancer cells after Etoposide treatment for 48 hours.** Relative *RAI2* transcript levels were determined by normalizing to the reference gene RPLPO using the ΔΔCₜ method.
**Figure 11****: Bioinformatical analyses of synergistically or antagonistically expressed genes in relation to RAI2. A)** SAM-analysis (Significance Analysis of Microarrays) of large published breast and lung cancer data sets identified several genes co-expressed with RAI2, mainly involved in the immediate early growth response or genes which are essential for the progression from G2-phase into mitosis. B) Expression of key regulators of cell cycle and proliferation in relation to RAI2.
**Figure 12****: RAI2 is not involved in the cell cycle regulation under non-stress conditions.** Synchronized MDA-MB-231 cells (12A) show no increase in RAI2 expression (12B) at any cell cycle phase under normal conditions.
**Figure 13****: Assessment of senescent and apoptotic cells after cellular stress in RAI2 mutant and overexpressing MDA-MB-231 cell lines.** Constitutive expression of the wild type RAI2 protein is leading to a considerable retardation of cell proliferation (13C) and increase in senescent cells (13A+B) but no increase in apoptosis (13D). This effect can not be seen with the mutated RAI2-cDNA-sequence and thus the interaction of RAI2 with CTBP2 is essential for the biological function of the RAI2 protein.
**Figure 14****: Generation of RAI2 mutants and Co-immune precipitation analysis with different mutant RAI2 expression constructs.** A) 3 different mutants were constructed by introducing mutations in the potential CTBP2 binding sites in RAI2. B) Expression of the different RAI2 constructs in western blot C) The mutation of both tandem binding motifs disrupts the interaction between RAI2 and CTBP2 completely.
**Fig. 15****: A summary of up- and downregulated genes among BM positive patients of combined analysis of tumors from breast, lung and colorectum,** Table A. * Upregulated=BM positiveBM negative, downregulated=BM negativeBM positive; ** false discovery rate; NS= non significant, NA=not available

### Examples

### Example 1: Association of early metastatic spread of primary epithelial tumors to BM with specific gene expression patterns

As a marker for early spread, DTC (disseminated tumor cells) in bone marrow (BM) were assessed. Breast, lung and colon carcinomas were selected as models for three major types of epithelial tumors that spread early to distant organs with a common high affinity to BM, but show a substantial difference in their ability to form overt metastases in BM. While overt bone/BM metastases are frequent in breast and lung cancer, they are rare in colon cancer.

Special emphasis was set in sample collection. Tumor and BM samples were collected from three different tumor entities (breast, colorectal and lung cancer). The samples were divided into two groups based on their BM status: (A) BM-negative and (B) BM-positive samples. All samples were from early stage primary tumors and matched for the main histological parameters between groups A and B. For all patients, the presence of DTC in BM and histopathological and clinical information on the patients, including survival data, was collected and stored in a central database.

For example, 30 primary breast cancer patients without lymph node metastases were investigated; divided in 15 patients with and 15 without BM micrometastasis. All tumors were of early stage (pT=1-2 and pN=0, M=0), hormone receptor positive and ductal carcinomas. The BM status was assessed by immunostaining of BM samples with anti pan-cytokeratin antibody A45-B/B3 (Micromet, Munich, Germany) and detection with an automated cell imaging system (Chromavision, Inc.). Depending on tumor cell homogeneity manual, macrodissection or laser microdissection was used to obtain a tumor cell content of at least 80%. Total RNA was extracted using RNeasy Micro Kit (Qiagen).

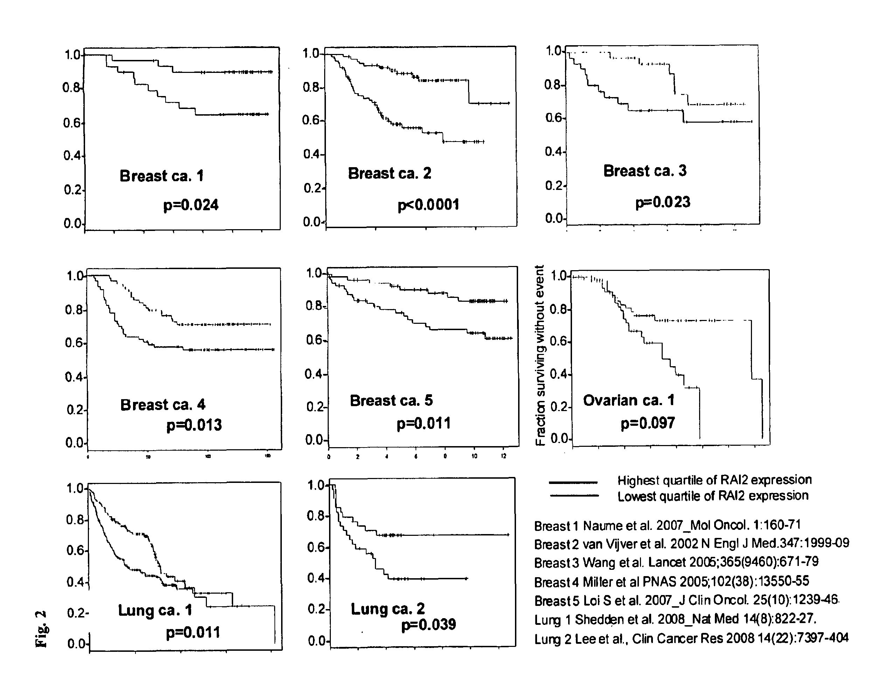

Altogether 114 cases (30 breast, 30 lung and 54 colorectal cancer cases) were analyzed using global gene expression profiling. In addition, for in-silico validation (Fig. 2), 8 publicly available gene expression data (5 breast data and 3 lung data sets) were retrieved from the Gene Expression Omnibus (GEO, www.geo.org) at GSE6532 (Loi et al. 2007), GEO at GSE8894 (Kim et al., 2007), GSE3494 (Miller et al 2005), GSE2034 (Wang et al. 2005) from CAARRAY database (NCI, US) at experiment ID 1015945236141280 (Shedden et al., 2008), or obtained from Rosetta Inpharmatics Inc. (http://www.rii.com/publications/2002/nejm.html; (van de Vijver et al., 2002).

Gene expression arrays were performed on the Affymetrix HGU133 Plus 2.0 GeneChips (Affymetrix, Santa Clara, CA) according to MIAME standards. cDNA-synthesis was performed using 50 ng of total RNA (Two-Cycle-Target Labeling Kit, Affymetrix) according to the GeneChip Expression Analysis Technical Manual. Gene expression data were normalized using gcrma (Wu et al., 2005). Customized chip description files (CDF) were used that re-map all probes from the Affymetrix GeneChips to ENSEMBL transcripts (Dai et al. 2005). To obtain transcripts that were significantly differentially expressed between BM positive and BM negative patients, the rank sum test described by Breitling et al. 2004 was used.

When comparing different cancer entities, the levels of expression of each transcript will be fundamentally different between them, and the magnitude of change between samples from patients with or without DTC in BM may be different as well. Therefore, for single tumor entities a cut-off for false discovery rate (fdr) was set as 0.2, whereas in the combined analysis top 25 up- or down-regulated genes were chosen for further analysis.

The rank sum test identified several genes possible associated with the BM status. Fig. 15 shows the significantly downregulated genes in the combined analysis of all three tumor entities as well as the breast and colorectal data alone. After correction for multiple testing the false discovery rates (fdr) were much lower among the downregulated genes (e.g. breast data 28 below 0.10) compared to the upregulated transcripts (8 below 0.10) indicating more genes downregulated among the BM positive patients than upregulated. Similarly, in the combined-analysis 7 of the 25 downregulated genes had a fdr below 0.20, whereas the best fdr for an upregulated gene was only 0.21. This is also reflected in the fact that the fold change differences between BM positive and BM negative was larger among the significantly down regulated genes compared to the upregulated genes (median: BMpos: 2.00 and BMneg: 1.43). Interestingly, whereas 10 out of the 25 downregulated and 3 of the 25 upregulated genes were also found significant in breast cancer analysis alone, only two genes were in common with the colorectal data and only 4 genes were found upregulated and 7 downregulated in the colorectal data.

In order to provide further evidence of the significance of DTC related genes, in-silico validations on the significant genes were performed using 5 breast and 3 lung cancer data sets. For colorectal data no expression data was available with follow up information. Using appropriately pre-processed gene expression values, samples in the validation sets were separated into high-expression and low expression groups by using the extreme quartiles. Differences in survival between these groups were determined by Kaplan-Meier estimates of survival, and by the log-rank test.

In breast cancer data, all genes with a fdr below 0.2 were tested for an association with overall-survival. RAI2 high expression was associated with significantly enhanced survival in all data sets (Fig. 1), whereas for RLN2, RERG, COX7A1 and ACO1, a significant association with improved survival in case of high expression could be found in at least in two data sets (Fig. 15). Among the four upregulated genes high expression of ABHD12 in two data sets and RNF43 in one data set was associated with worse survival.

In the combined analysis the expression of 19 out of the 50 genes was found significantly associated with overall survival at least in one data set (Fig. 15). *RAI2* was shown to be associated not only with all breast data sets but also with the lung data set making the finding highly interesting. In addition, the gene expression of five genes (downregulated: *PPP1R14A*, *RERG* and *RLN2*; upregulated: *INSIG1* and *NUFIP2*) was associated with survival in two breast data sets. Downregulation of *CYP2B7P1* and upregulation of *EROL1* were connected to worse survival in one breast and in one lung data set.

### Example 2: Expression of RAI2 and association to survival

In order to provide further evidence of the significance of the *RAI2* gene not only as a marker for early dissemination, but also as a prognostic factor, the inventors performed *in silico* validations in numerous large publicly available patient cohorts. Survival analysis was performed using five large published breast cancer datasets with 295 (van de Vijver et al., 2002), 123 (Naume et al., 2007), 666 (Loi et al., 2007), 286 (Wang et al., Lancet 2005;365:671-679) and 251 breast cancer patients (Miller et al PNAS 2005;102(38):13550-13555), where long follow-up data as well as expression data was available. In addition to the five large breast data sets, two additional lung cancer data sets consisting of 442 lung adenocarcinomas (Shedden et al. 2008) and 137 NSCLC patients (Kim et al., GEO at GSE8894) were used (Fig. 2).

Gene expression data on Affymetrix platforms were processed, using custom CDF that re-map probes to ENSEMBL transcripts (Dai et al 2005). For data on other platforms, probes were mapped based on sequence similarity to ENSEMBL transcripts, and expression summaries were calculated from probes that map to the same transcript. For this, probes that mapped to the same transcript were clustered by their Pearson correlation with a minimum correlation coefficient of 0.6. The largest cluster was used to calculate the expression summary by taking the median expression in each sample.

Using appropriately pre-processed gene expression values, samples in the validation sets were separated into high-expression and low expression groups by using the extreme quartiles. Differences in survival between these groups were determined by Kaplan-Meier estimates of survival, and by the log-rank test. All calculations were carried out in R version 2.7.2 (http://www.R-project.org) using packages from the Bioconductor project.

High RAI2 expression was shown to be associated with long time survival not only in all breast data sets, but also in the two lung data sets, making the discovery highly significant. The survival analyses in breast and lung cancer data sets are shown in Fig. 2. Interestingly, an oncomine data base search (www.oncomine.org) performed by the inventors showed that RAI2 is associated with the estrogen status in breast tumors. However, multivariate Cox proportional-hazard model on the five datasets showed that RAI2 survival information is independent of ER status as well as TNM status or grade in the 3 largest data sets (Table 1). These findings reveal a potential clinical relevance of the RAI2 gene and emphasize a putative active role of the RAI2 gene product in cancer progression making this gene highly interesting for a comprehensive functional analysis.

**Table 1. Multivariate Cox proportional-hazard ratio calculations for RAI2 expression in five 5 breast cancer data sets**

| | **n** | **coef** | **p-value** | **Hazard Ratio** | **95% CI** |
|---|---|---|---|---|---|
| **van de Vijver et al.** | 295 | 0,79 | 0,02 | 2,20 | 1.13-4.29 |
| **Loi et al.** | 666 | 0,75 | 0,03 | 2,11 | 1.10-4.07 |
| **Wang et al.** | 286 | 0,74 | 0,03 | 2,11 | 1.08-4.11 |
| **Miller et al** | 251 | 0,78 | 0,10 | 2,19 | 0.87-5.52 |
| **Naume et al.** | 125 | 1,13 | 0,19 | 3,09 | 0.58-16.38 |

### Example 3: Analysis of RAI2 expression on the transcript level in patient tumor tissue

In the screening analyses, microarray-based assays were used to reveal DTC-related gene expression patterns, which resulted in the identification of the *RAI2* gene as a potential metastasis suppressor protein. However, numerous publications have shown that microarray-based data need to be confirmed by independent methods. Therefore, the expression of *RAI2* was analyzed on the transcript level by quantitative RT-PCR analysis in an independent set of breast and lung cancer samples. A total of 30 primary breast tumors and 31 primary lung tumor samples were analyzed, which were separated according to their BM status. Additionally, 10 breast cancer and 20 lung cancer samples from brain metastasis were tested for *RAI2* expression. In order to analyze the RAI2 expression in tumor cells and not in the surrounding stroma, RNA was isolated from manually macrodissected cryosections using the RNeasy micro kit (Qiagen) as proposed by the manufacturer. Subsequently, 100 ng of RNA was used as template for the cDNA synthesis (cDNA synthesis kit; Fermentas). The mean relative RAI2 expression levels were obtained after quantitative RT-PCR with SYBR Green dye (Eurogentech) and normalization to the reference gene RPLPO and *universal* human reference (UHR).
RAI2 Forward 5'-ctcaatccgaatggcaatg-3' (SEQ ID NO: 1)
IRX-2 Reverse 5'-ctgaaagacgtgctgctcc-3' Amplicon 87 bp (SEQ ID NO: 2)
IRX2 Forward 5'-gctaccagaagcaaggacgaga-3' Amplicon 106 bp (SEQ ID NO: 3)
IRX-2 Reverse 5'-agtgatccgtgagcgagtcca-3' (SEQ ID NO: 4)
RPLPO-Forward 5'-acccagctctggagaaactgc-3' Amplicon 72 bp (SEQ ID NO: 5)
RPLPO-Reverse 5'-tgaggtcctccttggtgaaca-3' (SEQ ID NO: 6)

| | **µl Vol. pro sample** |
|---|---|
| **Total** | **15** |
| **2x Syber-Green-Mix** | **7.5** |
| **Rrimer For** | **0.4** |
| **Primer Rev** | **0.4** |
| **cDNA ( 5ng/µl)** | **5** |
| **H₂O** | **1.7** |

The cycling conditions were the following:

| **1X** | | | |
|---|---|---|---|
| **2 min** | 50°C, 10 min | 95°C | |

| **40x** | | | |
|---|---|---|---|
| 15 sec | 95°C, 30 sec | 60°C, 30 sec | 72°C |

| **Melting curve 1X:** | | | |
|---|---|---|---|
| 15 sec | 95°C, 15 sec | 60°C, 20 min heating | 60->95°C |

With this independent sample set, it could be confirmed that RAI2 expression is significantly downregulated in BM positive tumors (Fig. 1, 3 and 4). Furthermore, in line with the metastasis suppressor gene theory, relative low RAI2 expression was also detected in the analyzed brain metastases. In summary, these results from both primary tumor and metastasis samples provide evidence that the loss of *RAI2* gene expression is associated with an aggressive tumor phenotype and underline the putative role of *RAI2* as a metastasis suppressor gene.

### Example 4: Overexpression and cellular localization of RAI2

As RAI2 represents an almost uncharacterized protein, a model-system to study its molecular properties was established. It was first addressed whether the RAI2 protein may possibly be a pro-apoptotic molecule or might exhibit other cytotoxic properties, which could dramatically influence the subsequent functional characterization. The RAI2 protein was transiently over-expressed in HEK293 cells in order to reveal any influence on cellular viability. A RAI2 expression construct was established by cloning the RAI2-cDNA into the mammalian expression vector phCMV3 (AMS Biotechnology, Abingdon). The choice of this vector ensures a strong constitutive expression under the control of the CMV-promotor, and additionally a Hemagglutinin tag (HA) is attached to the c-terminus of the protein. 48 hours after transient transfection with the expression plasmid, the HEK293 cells neither showed a decreased viability nor morphologic alterations, whereas a strong recombinant protein expression was specifically detected by Western Blot analysis (Fig. 5 A) using commercial anti RAI2-antibody (Abgent, San Diego, CA). Hence, a direct cytotoxic effect resulting from RAI2 protein expression can be excluded, and stably transfected RAI2 overexpressing cell lines can be produced. These results are in line with the function of a metastasis suppressor protein.

An immunocytochemical staining using an HA-epitope-tag specific antibody (Sigma) in addition to the RAI2-specific antibody was also conducted. A nuclear localization of the RAI2 protein in the transiently transfected cells was revealed (Fig. 5 B and C). This result is confirmed by different bioinformatical approaches (PSORT, LOCtree), which also predict a nuclear localization sequence (NLS) at the c-terminus of the RAI2 gene product. The finding that RAI2 is a nuclear protein indicates that it might be involved in gene regulation processes.

### Example 5: RAI2 expression and biological function in cancer cell lines

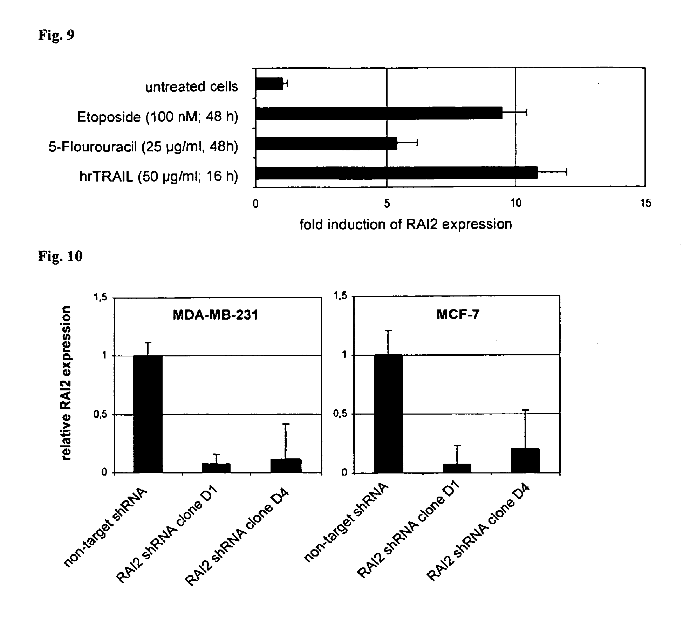

RAI2 expression in breast, colon and lung cancer cell lines was analyzed. Three well known cell lines were chosen for the constitutive over-expression of the RAI2 protein, namely MDA-MB-231 (breast), HT-29 (colon) and A-549 (lung). All theses three cell lines are known to be highly metastatic when implanted into immune deficient mice. It was found that these cell lines express the RAI2 gene at a very low level (shown for MDA-MB-231 in Fig. 9).

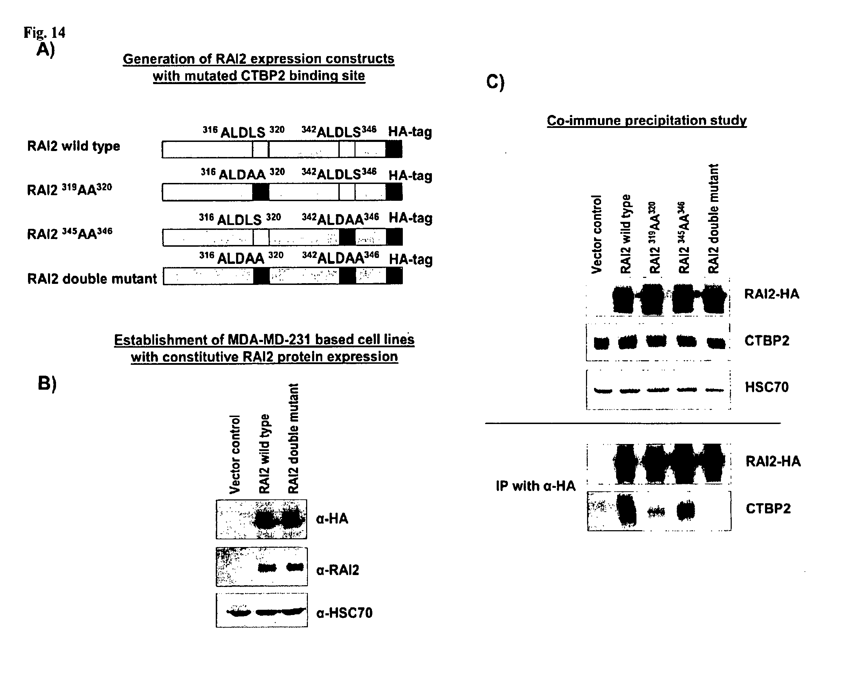

Thus these cell lines are well suited for studying a potential decreased or increased metastatic capacity via reconstitution or knockdown of RAI2 protein expression. For the establishment of the model system, a RAI2 expression plasmid was used as used for overexpression in MDA-MB-231 cells. The recipient cells were stably transfected with the expression plasmid using Lipofectamine 2000^{™} (Invitrogen) and individual clones were selected in the presence of G418. Subsequently positive cell clones showing stable over-expression were identified via Western Blot analysis using an epitope-specific monoclonal HA-antibody (Sigma-Aldrich) (Figure 14B). Cell clones owning high as well as moderate recombinant protein expression were chosen for the subsequent functional studies. Additionally a mock control for each of the chosen cell lines was established by using the bare expression plasmid for a stable transfection.

In cultured tumor-derived cell lines, like MDA-MB-231, the *RAI2* transcript could only be detected at very low levels. As shown in Fig. 9, the *RAI2* transcript was highly upregulated after the treatment of MDA-MB-231 with different apoptosis inducing compounds (Fig. 9).

In order to establish and analyse a sufficient *RAI2* knock-down in cultured cells, it is necessary to transfect the target cells with suitable siRNA sequences as well as to stimulate the cells subsequently. To generate a *RAI2* knock-down in MDA-MB-231 and MCF-7 breast cancer cells, the target cells were transduced with pLKO1 derived lentiviral supernatants (TRCN0000139927/clone D1 and TRCN0000145332/clone D4) and stable selection was performed with Puromycin (2 µg/ml) for four days. After that, the culture media were supplemented with 100 mM Etoposide and the mean relative *RAI2* knock down were obtained after 48 hours with quantitative RT-PCR. Normalization was performed to the reference gene RPLPO and cells which were transduced with a lentiviral non-target-shRNA particles. As shown in Fig. 10, a *RAI2* knock-down was successfully created in both shRNA-transduced cell lines.

To enable the surveillance of individual metastasis in the *in vivo* studies, the manipulated cells may be labeled with the firefly luciferase reporter protein by the use of a lentiviral gene transfer. For this propose the Luc2 Protein (Promega) may be used as the labeling molecule. This protein has been optimized for more efficient expression and codon usage in mammalian cells. In order to generate infectious particles, the lentiviral reporter plasmid is co-transfected together with the packaging plasmids psPAX2 and pMD2.G into HEK293T cells. The achieved lentiviral particles are afterwards used to transduce the recipient cells.

It is tested whether the over/under expression of RAI2 in the manipulated cells can influence cellular behavior in different processes important in carcinogenesis. The cells are analyzed regarding an altered potential of their proliferation, motility and invasiveness using different *in vitro* assays.

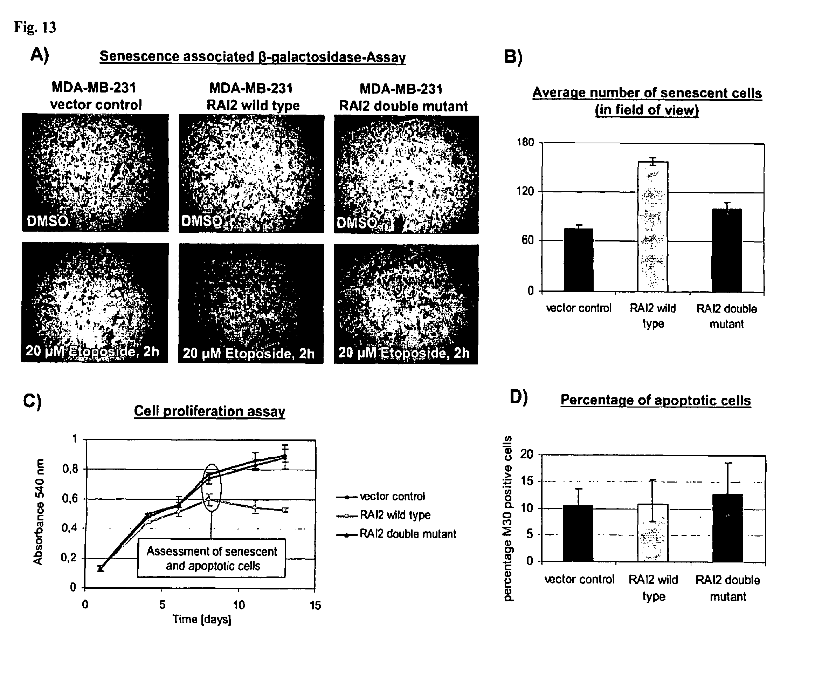

It is investigated whether RAI2 over/underexpressing cells show an altered cell proliferation in comparison to the parental and the mock control cell line. The proliferation rates of the cells can be compared via colorimetric analysis. It can be seen if the manipulated cells show changed anchorage independent growth properties in soft-agar. In this assay the cells are embedded in cell culture media containing 0.5% Agar. After two weeks of cultivation the number and dimension of colonies is determined and compared between different cells (Fig. 13C).

### Example 6. Assessment of genes which are synergistically or antagonistically expressed in RAI2 expressing tumors

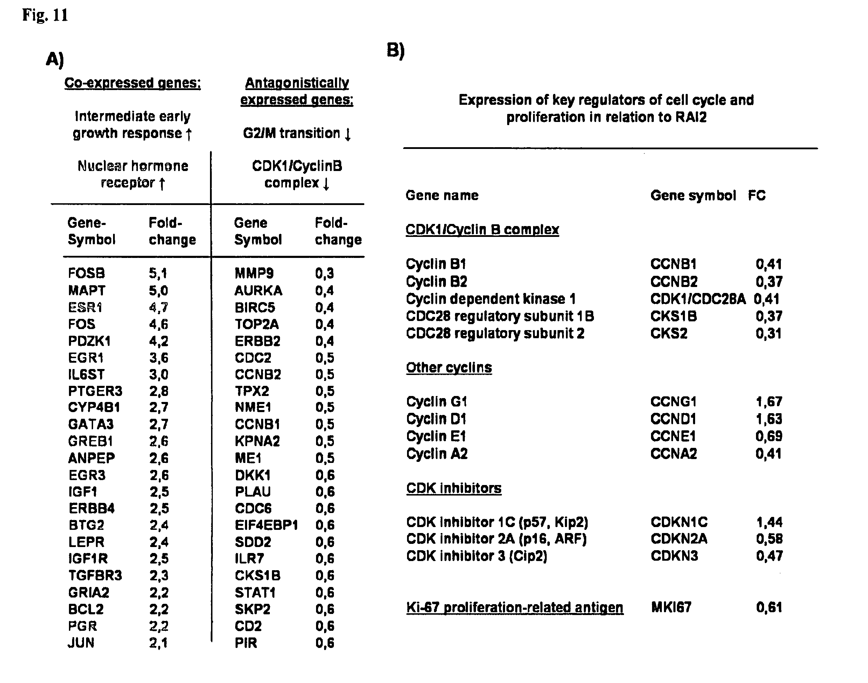

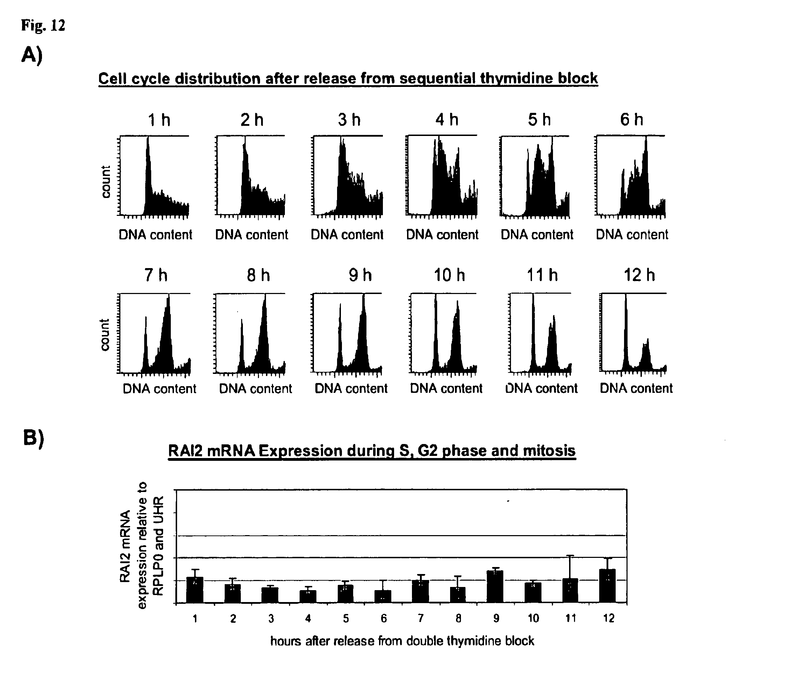

The biological function of the RAI2 gene product within adult and cancerous tissues remains nearly uncharacterized. With the purpose to obtain first evidence about its biological function in tumor tissue we performed a series of bioinformatical analyses to discover genes which are synergistically or antagonistically expressed in relation to RAI2. Based on a SAM-analysis (Significance Analysis of Microarrays) of the combination of one large published breast cancer and one lung cancer data set we were able to identify several genes whose expression correlates with RAI2 expression (Fig. 11). Most strikingly we discovered that RAI2 is co-expressed with several genes of the immediate early growth response. This indicates that the RAI2 gene is expressed within proliferating tissue. On the other hand we found that the expression of many genes which are essential for the progression from G2-phase into mitosis are significantly lower in RAI2 expressing tumors (Fig. 11A). Because the expression of these genes is tightly regulated within the cell cycle we assumed that RAI2 expression may also be cell cycle dependent. In order to monitor a possible cell cycle dependent regulation of RAI2 expression we synchronized MCF-7 cells at G1/S-junction by a sequential thymidine block. Since we could not detect any alterations of RAI2 expression after the release from thymidine block we conclude that correlation between high RAI2 and low expression of G2/M-genes can be seen in as a result of a cell cycle arrest in cells which suffer diverse cellular stress (Fig. 12). To validate this implication we performed another SAM-Analysis with a large published breast cancer data set and revised in particular to expression of important cell cycle and proliferation regulating genes. Thus, we could show that expression of Cylin D is notably raised in RAI2 expressing tumors whereas the expression of Cyclin A, B and E as well as MKI67 is considerably lowered (Fig. 11B). This indicates that RAI2 becomes indeed only induced under stressing conditions in course of a cell cycle arrest as we have shown by treating different cell lines with different stress inducing compunds (Fig. 9).

### Example 7. Constitutive expression of the RAI2 protein leads to increased survival of MDA-MB-231 breast cancer cells after treatment with genotoxic compounds

By systematic mapping of protein-protein interactions it has already been shown that the RAI2 gene product interacts with the transcriptional corepressor CTBP2 (Rual et al. 2005). Experiments performed by the inventors are in line with this hypothesis, showing, e.g., nuclear localization (Fig. 5). However, neither have the direct interacting of the RAI2 and CTBP2 proteins ever been validated with independent methods so far nor have the interacting protein domains been identified. We were able to identify a non-consensus bipartite CTBP2 binding site in the primary peptide sequence of the RAI2 protein. In order to show whether this binding site is in fact crucial for the known protein interaction we performed a co-immune precipitation analysis with different RAI2 expression construct which contain distinct amino acid substitutions in the proposed binding sites (Fig. 14A). By doing this we could show that the mutation of both tandem binding motifs disrupts the interaction between RAI2 and CTBP2 completely (Fig. 12C). Subsequently in order to show that the direct association to CTBP2 is essential for the function of RAI2, we inserted the mutated RAI2-cDNA-sequence as well as the constitutively expressed wild-type RAI2 into a retroviral expression vector and transduced MDA-MD-231 breast cancer cells with both constructs and a vector control (no RAI2 sequence). Successful transduced cells were afterwards selected in presence of puromycin. (Fig. 14A and B).

In order to examine possible consequences on cellular properties which were either associated with reconstitution of RAI2 expression in MDA-MB-231 cells or disruption of RAI2/CTBP2 interaction we conducted a series of experiments (Fig. 13). Hence, all three cell lines were subjected to a short pulse of chemotherapeutical treatment. Afterwards the cellular proliferation was monitored for two weeks. In addition we determined the percentage of apoptotic cells as well as the relative proportion of senescent cells one week after the chemotherapeutical treatment. By doing this we could show that constitutive expression of the wild type RAI2 protein is leading to a considerable retardation of cell proliferation (13C). This effect is combined by the increased occurrence of growth arrested/scenescent cells (13B), while no differences in the percentage of apoptotic cells can be detected. (13D). As this effect can not be seen in MDA-MB-231 cells which were transduced with the mutated RAI2-cDNA-sequence we could show that the interaction of RAI2 with CTBP2 is essential for the biological function of the RAI2 protein. Additionally the inventors can show with this experiment that the RAI2 protein determines cellular fate after the exposure to genotoxic stress by modulating gene expression and thus involved in establishment of scenescence.

### Publications

Alix-Panabières C, et al., Detection and characterization of putative metastatic precursor cells in cancer patients. Clin Chem. 2007 Mar;53(3):537-9.
Balic et al., Most early disseminated cancer cells detected in bone marrow of breast cancer patients have a putative breast cancer stem cell phenotype. Clin Cancer Res. 2006 Oct 1;12(19):5615-21.
Bhattacharjee et al. Classification of human lung carcinomas by mRNA expression profiling reveals distinct adenocarcinoma subclasses. PNAS 2001;98(24):13790-13795
Boucharaba et al. 2006. The type 1 lysophosphatidic acid receptor is a target for therapy in bone metastases. PNAS 103(25):9643-8.
Braun S, et al. (2005) A pooled analysis of bone marrow micrometastasis in breast cancer. N Engl J Med 353: 793-802.
Braun S, et al. (2000) Cytokeratin-positive cells in the bone marrow and survival of patients with stage I, II, or III breast cancer. N Engl J Med 342: 525-33.
Breitling et al. 2004 FEBS Lett. 2004 Aug 27;573(1-3):83-92
Chinnadurai et al.. The transcriptional corepressor CtBP: a foe of multiple tumor suppressors. Cancer Res. 2009 Feb 1;69(3):731-4.
Dai et al. Nucleic Acids Res. 2005 Nov 10;33(20):e175.
Dango et al., Elevated expression of carcinoembryonic antigen-related cell adhesion molecule 1 (CEACAM-1) is associated with increased angiogenic potential in non-small-cell lung cancer. Lung Cancer. 2008 Jun;60(3):426-33.
Essers, M.A., et al. (2009). IFNalpha activates dormant haematopoietic stem cells in vivo. Nature 458, 904-908.
Eccles SA et al., Metastasis: recent discoveries and novel treatment strategies. Lancet. 2007 May 19;369(9574):1742-57
Fehm et al., A concept for the standardized detection of disseminated tumor cells in bone marrow from patients with primary breast cancer and its clinical implementation.Cancer. 2006 Sep 1;107(5):885-92
Finak et al., Differential gene expression in breast cancer cell lines and stroma-tumor differences in microdissected breast cancer biopsies revealed by display array analysis. Int J Cancer. 2002 Jul 10;100(2):172-80.
Finak et al. Stromal gene expression predicts clinical outcome in breast cancer Nat Med. 2008. 14:518-27.
Köllermann et al., Prognostic significance of disseminated tumor cells in the bone marrow of prostate cancer patients treated with neoadjuvant hormone treatment. J Clin Oncol. 2008 Oct 20;26(30):4928-33. Epub 2008 Sep 15.
Janni et al., The persistence of isolated tumor cells in bone marrow from patients with breast carcinoma predicts an increased risk for recurrence. Cancer. 2005 Mar 1; 103(5):884-9 1.
Laurenti, E., et al. (2008). Hematopoietic stem cell function and survival depend on c-Myc and N-Myc activity. Cell Stem Cell 3, 611-624.
Lee et al., Clin Cancer Res 2008 Nov 15;14(22):7397-404
Li et al. 2006 Mol Cell Biol. 2006 Jun;26(11):4240-56.
Li et al. 2006. Anticancer Res. 2006 Sep-Oct;26(5A):3555-60
Loi S et al. 2007_J Clin Oncol. 25(10):1239-1246
Miller et al PNAS 2005;102(38):13550-13555
Molloy et al., Breast Cancer Res Treat. 2008 Nov;112(2):297-307
Naume et al. 2007_Mol Oncol. 1:160-171.
Niggemann B et al., Tumor cell locomotion: differential dynamics of spontaneous and induced migration in a 3D collagen matrix. Exp Cell Res. 2004 Aug 1;298(1):178-87.
Palmieri, et al. 2005. Medroxyprogesterone acetate elevation of Nm23-H1 metastasis suppressor expression in hormone receptor-negative breast cancer. J Natl Cancer Inst. 97(9):632-42.
Pantel and Brakenhoff, 2004. Dissecting the metastatic cascade. Nat Rev Cancer. 2004 Jun;4(6):448-56
Pantel K, et al B (2008) Detection, clinical relevance and specific biological properties of disseminating tumour cells. Nature Rev Cancer 8: 329-40.
Rual et al. Towards a proteome-scale map of the human protein-protein interaction network. Nature. 2005, 437:1173-8.
Stafford LJ, et al., Metastasis suppressors genes in cancer. Int J Biochem Cell Biol. 2008;40(5):874-91.
Shedden et al. 2008_Nat Med 14(8):822-827.
Solakoglu O, et al. (2002) Heterogeneous proliferative potential of occult metastatic cells in bone marrow of patients with solid epithelial tumors. Proc Natl Acad Sci U S A 99: 2246-51.
Tanaka et al.. Frequent methylation-associated silencing of a candidate tumor-suppressor, CRABP1, in esophageal squamous-cell carcinoma. Oncogene. 2007.26:6456-68
Titus et al., 2005. Cancer Res. 2005 Aug 15;65(16):7320-7.
van de Vijver MJ et al. 2002_N Engl J Med.347(25):1999-2009
Walpole et al., Identification and characterization of the human homologue (RAI2) of a mouse retinoic acid-induced gene in Xp22. Genomics. 1999 Feb 1;55(3):275-83.
Wang et al. Lancet 2005;365(9460):671-679
Wikman et al., Cancer micrometastasis and tumour dormancy. APMIS. 2008 Jul-Aug;116(7-8):754-70.
Wilson, A., and Trumpp, A. (2006). Bone-marrow haematopoietic-stem-cell niches. Nat Rev Immunol 6, 93-106.
Wilson, A., et al. (2008). Hematopoietic stem cells reversibly switch from dormancy to self-renewal during homeostasis and repair. Cell 135, 1118-1129.
Woelfle U, et al. (2003) Molecular signature associated with bone marrow micrometastasis in human breast cancer. Cancer Res 63: 5679-84.
Woelfle U, et al. (2005.) Influence of immunomagnetic enrichment on gene expression of tumor cells. J Transl Med. 2005 Mar 16;3(1):12.
Wrage M, et al. Genomic profiles associated with early micrometastasis in lung cancer: relevance of 4q deletion. Clin Cancer Res. 2009 Mar 1; 15(5): 1566-74.
van de Vijver MJ et al., A gene-expression signature as a predictor of survival in breast cancer. N Engl J Med. 2002 Dec 19;347(25):1999-2009.
US application No. 20100009357
US application No. 20100021424
US application No. 20090175844
US application No. 20080318234
US application No. 20080292546

## Claims

1. A method of determining the risk of survival of tumor cells in the bone marrow of a patient, comprising determining the expression of RAI2 (retinoid acid induced gene 2) in a tumor sample obtained from the patient.

2. The method of claim 1, wherein the tumor from which the sample is obtained is a primary tumor.

3. The method of any preceding claim, wherein the tumor is selected from the group comprising breast cancer and colorectal carcinoma.

4. The method of any preceding claim, wherein the expression of RAI2 is determined on the protein or RNA level.

5. The method of any preceding claim, wherein the expression of RAI2 is determined by RT-PCR, gene chip analysis, hybridization, ELISA, Western Blot, dot blot, immunofluorescent methods or FACS.

6. The method of any preceding claim, wherein the expression of RAI2 and IRX2 in the sample is determined.

7. The method of any preceding claim, wherein, additionally, the expression of one or more genes selected from the group comprising APM2, AQP1, CDO1, CYP2B7P1, PLAC9, PPP1R14A, RGS16, SCGB3A1, SERP2, FBXO15, HSD17B1, INHBB, IRX2, MAOB, RBP7, RERG, RLN2, ST3GAL1, ABCC8, ACO1, ANXA3, ASB13, CD44, COX7A1, FHL2, HSD11B2, LRRN2, MGST3, MPP2, NLRP2, PLIN, PPARG, PRKAR2B, REEP1, SCN4B, ST6GALNAC5, C1orf163, DDIT4, ENTPD7, ERO1L, FUT11, GNE, GUF1, HOXB13, INSIG1, LYCAT, ME1, NFKBIZ, NUFIP2, SPTLC2, TMEM14D, YEATS4, CPD, RNF43, ZNF518B, and ABHD12 in the sample is determined.

8. Use of a kit comprising means for determining the expression of RAI2 in a sample obtained from the patient for determining the risk of survival of tumor cells in the bone marrow.

9. Use of claim 8, wherein the means are selected from the group comprising an antibody to RAI2, primers capable of specifically hybridizing to and amplifying RAI2, and a probe capable of specifically hybridizing to RAI2.

## Patentansprüche

1. Verfahren zur Bestimmung des Risikos des Überlebens von Tumorzellen im Knochenmark eines Patienten, bei dem man die Expression von RAI2 (Retinolsäure-induziertes Gen 2) in einer vom Patienten erhaltenen Tumorprobe bestimmt.

2. Verfahren nach Anspruche 1, bei dem der Tumor, von dem die Probe erhalten wurde, ein primärer Tumor ist.

3. Verfahren nach einem der vorhergehenden Ansprüche, bei dem der Tumor ausgewählt ist aus der Gruppe bestehend aus Brustkrebs und kolorektalem Karzinom.

4. Verfahren nach einem der vorhergehenden Ansprüche, bei dem die Expression von RAI2 auf Proteinebene oder RNA-Ebene bestimmt wird.

5. Verfahren nach einem der vorhergehenden Ansprüche, bei dem die Expression von RAI2 mittels RT-PCR, Gen-Chip-Analyse, Hybridisierung, ELISA, Western-Blot, Dot-Blot, Immunofluoreszenz-Verfahren oder FACS bestimmt wird.

6. Verfahren nach einem der vorhergehenden Ansprüche, bei dem die Expression von RAI2 und IRX2 in der Probe bestimmt wird.

7. Verfahren nach einem der vorhergehenden Ansprüche, bei dem zusätzlich die Expression von einem oder mehreren Genen aus der Gruppe bestehend aus APM2, AQP1, CDO1, CYP2B7P1, PLAC9, PPP1R14A, RGS16, SCGB3A1, SERP2, FBXO15, HSD17B1, INHBB, IRX2, MAOB, RBP7, RERG, RLN2, ST3GAL1, ABCC8, ACO1, ANXA3, ASB13, CD44, COX7A1, FHL2, HSD11B2, LRRN2, MGST3, MPP2, NLRP2, PLIN, PPARG, PRKAR2B, REEP1, SCN4B, ST6GALNAC5, Clorf163, DDIT4, ENTPD7, ERO1L, FUT11, GNE, GUF1, HOXB13, INSIG1, LYCAT, ME1, NFKBIZ, NUFIP2, SPTLC2, TMEM14D, YEATS4, CPD, RNF43, ZNF518B und ABHD12 in der Probe bestimmt wird.

8. Verwendung eines Kits, der Mittel zur Bestimmung der Expression von RAI2 in einer vom Patienten erhaltenen Probe umfasst, zur Bestimmung des Risikos des Überlebens von Tumorzellen im Knochenmark.

9. Verwendung gemäß Anspruch 8, wobei die Mittel ausgewählt sind aus der Gruppe umfassend einen Antikörper gegen RAI2, Primer, die in der Lage sind, spezifisch an RAI2 zu hybridisieren und dieses zu amplifizieren, und eine Sonde, die in der Lage ist, spezifisch an RAI2 zu hybridisieren.

## Revendications

1. Procédé destiné à déterminer le risque de survie de cellules tumorales dans la moelle osseuse d'un patient, comprenant une étape consistant à déterminer l'expression de RAI2 (gène 2 induit par l'acide retinoïque) dans un échantillon de tumeur obtenu en provenance du patient.

2. Procédé selon la revendication 1, dans lequel la tumeur à partir de laquelle est obtenu l'échantillon, est une tumeur primaire.

3. Procédé selon l'une quelconque des revendications précédentes, dans lequel la tumeur est sélectionnée dans le groupe constitué par le cancer du sein et le carcinome colorectal.

4. Procédé selon l'une quelconque des revendications précédentes, dans lequel l'expression de RAI2 est déterminée au niveau protéine ou ARN.

5. Procédé selon l'une quelconque des revendications précédentes, dans lequel l'expression de RAI2 est déterminée par RT-PCR, analyse d'un morceau de gène, hybridation, ELISA, transfert de Western, transfert en point, procédés d'immunofluorescence ou FACS.

6. Procédé selon l'une quelconque des revendications précédentes, dans lequel est déterminée l'expression de RAI2 et d'IRX2 dans l'échantillon.

7. Procédé selon l'une quelconque des revendications précédentes, dans lequel, de plus, est déterminée dans l'échantillon l'expression d'un ou de plusieurs gènes sélectionnés dans le groupe constitué par APM2, AQP1, CDO1, CYP2B7P1, PLAC9, PPP1R14A, RGS16, SCGB3A1, SERP2, FBXO15, HSD17B1, INHBB, IRX2, MAOB, RBP7, RERG, RLN2, ST3GAL1, ABCC8, ACO1, ANXA3, ASB13, CD44, COX7A1, FHL2, HSD11B2, LRRN2, MGST3, MPP2, NLRP2, PLIN, PPARG, PRKAR2B, REEP1, SCN4B, ST6GALNAC5, C1orf163, DDIT4, ENTPD7, ERO1L, FUT11, GNE, GUF1, HOXB13, INSIG1, LYCAT, ME1, NFKBIZ, NUFIP2, SPTLC2, TMEM14D, YEATS4, CPD, RNF43, ZNF518B et ABHD12.

8. Utilisation d'un kit comprenant des moyens destinés à déterminer l'expression de RAI2 dans un échantillon obtenu en provenance du patient de façon à déterminer le risque de survie des cellules tumorales dans la moelle osseuse.

9. Utilisation selon la revendication 8, dans lequel les moyens sont sélectionnés dans le groupe constitué par un anticorps à RAI2, des amorces capables de s'hybrider de manière spécifique à RAI2 et de l'amplifier, et une sonde capable de s'hybrider de manière spécifique à RAI2.
